(19) Europäisches Patentamt
European Patent Office
Office européen des brevets

(11) Publication number: **0 237 952 B1**

# (12) EUROPEAN PATENT SPECIFICATION

(45) Date of publication of patent specification: **30.10.91**

(51) Int. Cl.⁵: **C07D 223/22**

(21) Application number: **87103545.7**

(22) Date of filing: **11.03.87**

The file contains technical information submitted after the application was filed and not included in this specification

## (54) Process for the preparation of 5H-Dibenzo-(b, f)-azepine.

(30) Priority: **14.03.86 FI 861057**

(43) Date of publication of application:
**23.09.87 Bulletin 87/39**

(45) Publication of the grant of the patent:
**30.10.91 Bulletin 91/44**

(84) Designated Contracting States:
**AT BE CH DE ES FR GB GR IT LI LU NL SE**

(56) References cited:
**US-A- 3 074 931**

**CHEMISCHE BERICHTE, vol. 93, no. 2, 29th February 1960, pages 392-397, Weinheim, DE; R. HUISGEN et al.: "2.3;6.7-Dibenzo-azepin"**

(73) Proprietor: **ORION-YHTYMÄ OY FERMION**
**P.O. Box 28**
**SF-02101 Espoo(FI)**

(72) Inventor: **Mannonen, Matti Tapani**
**Ristolantie 20 B 24**
**SF-00300 Helsinki(FI)**
Inventor: **Hilden, Leif**
**Metsätie 9**
**SF-04130 Nikkilä(FI)**

(74) Representative: **Weinhold, Peter, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz**
**Siegfriedstrasse 8**
**W-8000 München 40(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 237 952 B1

## Description

The invention relates to a new process for the preparation of 5H-Dibenzo-(b, f)-azepine, or iminostilbene of the formula (I) above.

Iminostilbene is used as an intermediate for the preparation of pharmacologically valuable substances such as opipramol or carbamazepine:

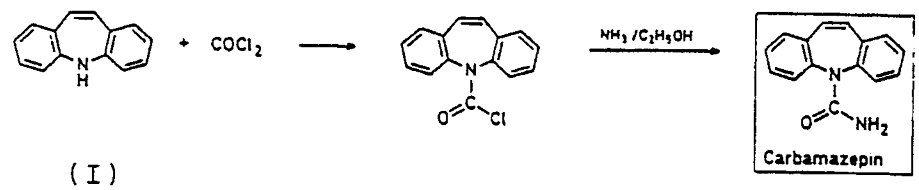


(I)

The iminostilbene (I), prepared according to the invention in question, has earlier been produced in another way.

Iminostilbene has thus been earlier prepared from iminodibenzyl, by using conventional chemical processes, by acylation, bromination, dehydrobromination and deacylation.

This method of synthesis in four steps is, however, very laborious and puts a high demand on production capacity and is therefore uneconomical.

According to US Pat.No. 3.074.931, the iminostilbene is produced from iminodibenzyl by catalytic dehydrogenation. By this process, iminodibenzyl in gaseous phase is forced through a glass column packed with a dehydrogenation catalyst at such a high temperature as 350-620 °C.

Sulfur, selenium or noble metals such as palladium, ruthenium, iridium, rhodium or osmium, together with a support such as carbon, barium sulfate or silica gel, could act as suitable catalysts.

According to the abovementioned patent, the reaction could alternatively be performed in a vessel by heating iminodibenzyl, either plain iminodibenzyl as such or together with a diluent as diphenyl, diphenyloxide or dimethylaniline at a temperature of 175-300 °C by using one of the abovementioned catalysts. The product is isolated by fractional crystallization or chromatographically.

The disadvantage on a technical scale of the first method is, however, as described in the abovementioned patent, the need for a very expensive equipment because of the high reaction temperatures.

The alternative process, according to the same patent, is only shortly presented and according to this method, hydrogen acceptors, which are necessary in order to achieve a more complete reaction, are not used at all. Moreover, this process does not at all appear in the claims or in the examples of this patent, because of difficulties with the isolation of the iminostilbene from the reaction mixture.

In US Pat.No. 3.449.324 a process is disclosed, in which iminostilbene is prepared by forcing iminodibenzyl in gas phase over a catalyst comprising of 35-70% $Fe_2O_3$, 1,5-3,0% $Cr_2O_3$, 10-15% CaO and $K_2CO_3$ combined with a carrier substance such as carbon. The reaction temperature is about400-500 °C. Gases such as for instance nitrogen, ammonia, argon, steam etc., could be used as diluents. The iminostilbene thus produced by this process is recovered from the gas phase, which is later condensed by the aid of spraying water.

The above described method has, according to the inventor, the advantage, that it is possible to use cheap catalysts, while on the other hand the yield of the desired substance is only about 60% after the first reaction cycle. Moreover, the first reaction mixture contains about 12-20% unreacted starting material. By recycling this reaction mixture over and over again, the yield could be raised, however.

The total yield varies between 55% and 80% as calculated from the amount of the starting material.

According to the German patent, DBP 1.545.736, iminostilbene is produced by dehydrogenating iminodibenzyl in gas phase at high temperatures, of about 380-620 °C in the presence of catalysts such as cobalt, nickel, vanadium, chromium, copper, molybdenum or tungsten. The above-mentioned metals could act as catalysts as such or together with supports as silica gel, aluminium oxide, silicon or asbestos.

The disadvantage of this method is a very high reaction temperature, which will be very expensive as to the heat economy of the process and the equipment. At the same time undesired decomposition products are produced. According to the method described above, hydrogen acceptors are not used. With this method, about 60% of the iminodibenzyl is transformed into iminostilbene.

Thus, object of the invention is to make available a process for the preparation of iminostilbene by catalytic dehydrogenation of iminodibenzyl avoiding these disadvantages.

Subject matter of the invention is a process for the preparation of 5H-Dibenzo-(b, f)-azepine, or iminostilbene of formula (I),

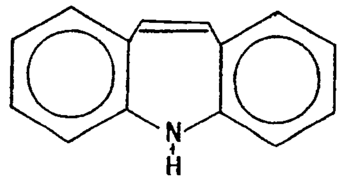

I

by catalytic dehydrogenation of 10,11-Dihydro-5H-dibenzo-(b, f)-azepine or iminodibenzyl of formula (II),

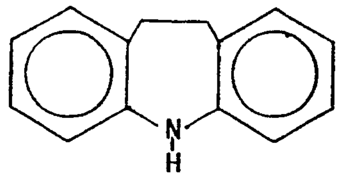

II

the dehydrogenation being performed in one step in liquid phase in the presence of a catalyst and a hydrogen acceptor at elevated temperature, characterized in that nitrotoluene is used as hydrogen acceptor and that the dehydrogenation is carried out at a temperature of 180-280° C.

As catalysts preferably noble metal catalysts, such as platinum or palladium on activated carbon or metaloxide catalysts, such as $Fe_2O_3$ or $Cr_2O_3$ are used. The reaction mixture may contain an excess of the hydrogen acceptor. The preferred temperature for the dehydrogenation reaction is 210-250° C.

The product is recovered by filtration after filtering off the catalyst and the filtrate is cooled.

According to an embodiment of the present invention, the iminostilbene is precipitated from the reaction mixture by the aid of methanol, ethanol, iso-propanol, hexane or heptane after the catalyst has been removed.

An advantage of the present invention is that the reaction is performed by the aid of the hydrogen acceptor in one step and in a liquid phase at a considerably low temperature, whereafter the recovery of the product is made simply by first filtering off the catalyst from the hot solution and, after cooling of the filtrate, by filtering the mother liquid.

Any special demands on the equipment are not needed because of the low reaction temperature, and the process is easy and cheap to perform even on a technical scale. The time for the reaction to occur is short, and as a consequence, the decomposition of the starting material and the product during the process is very low. Thus the end product does not contain any notable amounts of impurities. The yield of the reaction is high.

The invention is explained by the following examples.

Example I.

To a mixture containing 30 g iminodibenzyl and 90 ml 2-nitrotoluene, l,8 g 5% palladium on activated carbon catalyst is added. The mixture is heated for 3-5 hours at 180-230° C under reflux. The catalyst is filtered off from the hot solution whereafter 90 ml of methanol is added to the filtrate. The filtrate is cooled and the crystallized iminostilbene is filtered from the mother liquid. The yield of the dried iminostilbene is 24,3 g (82% of theoretical), m.p. 196-197° C.

Example 2.

To a mixture containing 30 g iminodibenzyl and 60 ml 3-nitrotoluene, l,8 g 5% palladium on activated carbon catalyst is added. The mixture is heated for 3-5 hours at 210-240° C under reflux. The catalyst is filtered off from the hot solution whereafter 90 ml iso-propanol is added to the filtrate. The filtrate is cooled and the crystallized iminostilbene is filtered from the mother liquid. The yield of iminostilbene is 23,2 g (78% of theoretical), m.p. 195-197° C.

Example 3.

To a mixture containing 30 g iminodibenzyl and 60 ml 2-nitrotoluene, 1,8 g 3% platinum on activated carbon catalyst is added. The mixture is heated for 3-5 hours at 210-240 °C under reflux. The catalyst is filtered off from the hot solution. The filtrate is cooled and the crystallized iminostilbene is filtered from the mother liquid. The yield or iminostilbene is 22,2 g (77% of theoretical), m.p. 195-197 °C.

## Claims

1. A process for the preparation of 5H-Dibenzo-(b, f)-azepine, or iminostilbene of formula (I),

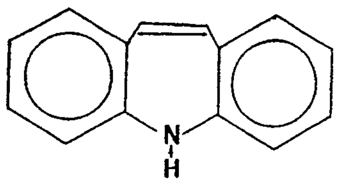

I

by catalytic dehydrogenation of 10,11-Dihydro-5H-dibenzo-(b, f)-azepine or iminodibenzyl of formula (II),

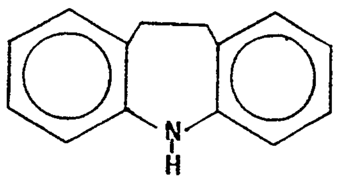

II

the dehydrogenation being performed in one step in liquid phase in the presence of a catalyst and a hydrogen acceptor at elevated temperature, characterized in that nitrotoluene is used as hydrogen acceptor and that the dehydrogenation is carried out at a temperature of 180-280 °C.

2. A process according to the claim 1, characterized in that the dehydrogenation is performed at a temperature of 210-250 °C.

3. A process according to one or more of the claims 1-2, characterized in that as catalysts, noble metal catalysts on activated carbon or metaloxide catalysts, are used.

4. A process according to claim 3, characterized in that the noble metal catalysts are platinum or palladium on activated carbon.

5. A process according to claim 3, characterized in that the metaloxide catalysts are $Fe_2O_3$ or $Cr_2O_3$.

6. A process according to one or more of the claims 1-5, characterized in that after the catalyst has been removed, the iminostilbene is precipitated from the reaction mixture by the aid of methanol, ethanol, iso-propanol, hexane or heptane.

## Revendications

1. Procédé de préparation de 5H-dibenzo-(b,f)-azépine, ou iminostilbène de formule (I)

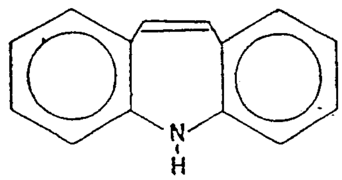

I

par déshydrogénation catalytique de 10,11-dihydro-5H-dibenzo-(b,f)-azépine ou iminodibenzyle de formule (II)

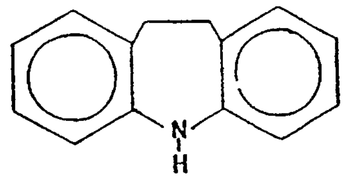

II

la déshydrogénation étant effectuée en une étape en phase liquide en présence d'un catalyseur et d'un accepteur d'hydrogène à une température élevée, caractérisé en ce que du nitrotoluène est utilisé comme accepteur d'hydrogène et que la déshydrogénation est réalisée à une température de 180 à 280 °C.

2. Procédé suivant la revendication 1, caractérisé en ce que la déshydrogénation est réalisée à une température de 210 à 250 °C.

3. Procédé suivant la revendication 1 ou la revendication 2, caractérisé en ce qu'on utilise en tant que catalyseurs, des catalyseurs impliquant un métal noble sur un carbone actif ou des catalyseurs à base d'oxyde métallique.

4. Procédé suivant la revendication 3, caractérisé en ce que les catalyseurs impliquant un métal noble comprennent du platine ou du palladium sur un carbone actif.

5. Procédé suivant la revendication 3, caractérisé en ce que les catalyseurs à base d'oxyde métallique sont $Fe_2O_3$ ou $Cr_2O_3$.

6. Procédé suivant l'une quelconque des revendications 1 à 5, caractérisé en ce qu'après avoir éliminé le catalyseur, on fait précipiter l'iminostilbène à partir du mélange réactionnel à l'aide de méthanol, d'éthanol, d'iso-propanol, d'hexane ou d'heptane.

**Patentansprüche**

1. Verfahren zur Herstellung von 5H-Dibenzo-(B,F)-azepin oder Iminostilben der Formel (I)

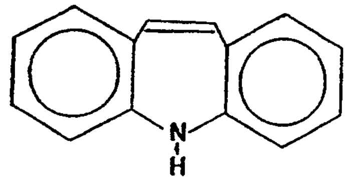

(I)

durch katalytische Dehydrierung von 10,11-Dihydro-5H-dibenzo-(B,F)-azepin oder Iminodibenzyl der Formel (II)

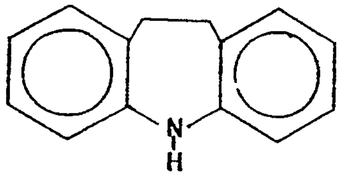

(II) ,

wobei die Dehydrierung in einem Schritt in flüssiger Phase in Gegenwart eines Katalysators und eines Wasserstoffakzeptors bei erhöhter Temperatur durchgeführt wird, dadurch gekennzeichnet, daß Nitrotoluol als Wasserstoffakzeptor verwendet und die Dehydrierung bei einer Temperatur von 180 bis 280 °C durchgeführt wird.

2. Verfahren gemaß Anspruch 1, dadurch gekennzeichnet, daß die Dehydrierung bei einer Temperatur von 210 bis 250 °C durchgeführt wird.

3. Verfahren gemäß einem oder mehreren der Ansprüche 1 bis 2, dadurch gekennzeichnet, daß als Katalystoren Edelmetallkatalysatoren auf Aktivkohle oder Metalloxidkatalysatoren verwendet werden.

4. Verfahren gemäß Anspruch 3, dadurch gekennzeichnet, daß die Edelmetallkatalysatoren Platin oder Palladium auf Aktivkohle sind.

5. Verfahren gemäß Anspruch 3, dadurch gekennzeichnet, daß die Metalloxidkatalysatoren $Fe_2O_3$ oder $Cr_2O_3$ sind.

6. Verfahren gemäß einem oder mehreren der Ansprüche 1 bis 5, dadurch gekennzeichnet, daß nach der Entfernung des Katalysators das Iminostilben aus der Rekationsmischung mit Hilfe von Methanol, Ethanol, Isopropanol, Hexan oder Heptan ausgefällt wird.